# EUROPEAN PATENT APPLICATION

(11) **EP 3 173 791 A1**
(43) Date of publication of application: **31.05.2017**
(21) Application number: 15824079.6
(22) Date of filing: 16.03.2015
(51) Int. Cl.: G01N 33/68, G01N 33/48

(54) **METHOD FOR DETERMINING PANCREATIC DISEASE**

(30) Priority: 23.07.2014 JP 2014149942
(71) Applicant: OLYMPUS CORPORATION, Hachioji-shi Tokyo 192-8507 (JP)
(72) Inventor: SANUKI Hiromi, Tokyo 192-8507 (JP); NAKATA Mari, Tokyo 192-8507 (JP); TAKEYAMA Tetsuhide, Tokyo 192-8507 (JP)
(74) Representative: Schicker, Silvia
(86) International application number: PCT/JP2015/057681
(87) International publication number: WO 2016/013248

(57) **Abstract**

A method of determining pancreatic disease of the present invention is a method of determining whether an IPMN patient has pancreatic disease, the method including: a first step of measuring a concentration of S100P in duodenal juice of the IPMN patient; and a second step of determining at least one of IPMN malignancy and the presence of pancreatic cancer in the IPMN patient based on determination of whether the concentration of the S100P is equal to or greater than a predetermined cutoff value.

## Description

### [Technical Field]

The present invention relates to a method of determining pancreatic disease.

Priority is claimed on Japanese Patent Application No. 2014-149942, filed July 23, 2014, the content of which is incorporated herein by reference.

### [Background Art]

An intraductal papillary mucinous neoplasm (IPMN) is a tumor that grows within pancreatic ducts and forms cysts. An IPMN has various stages. In some stages, an IPMN has progressed to a malignant stage and the IPMN invades the pancreatic parenchyma that is a part responsible for the main function of the pancreas. If a malignant IPMN can be identified earlier, since it is possible to perform an appropriate treatment before the disease progresses, a patient diagnosed with an IPMN is usually subjected to regular examination (follow-up examination).

However, a method through which a malignant IPMN can be conveniently detected is not currently provided. Although the IPMN itself can be easily detected using diagnostic imaging such as CT and MRI, advanced techniques about interpretation of images are necessary to identify invasion of an IPMN into the pancreatic parenchyma earlier using diagnostic imaging. Therefore, facilities in which invasion of the IPMN into the pancreatic parenchyma can be identified earlier are limited to facilities employing technicians with advanced interpretation techniques.

As a method of identifying invasion of an IPMN into the pancreatic parenchyma, methods of cannulating the pancreatic ducts, collecting pancreatic juice, and examining cells are provided in addition to the aforementioned method. However, there are problems, for example, cannulating the pancreatic ducts is work that requires highly technical capabilities and there is a risk of causing acute pancreatitis due to the diagnosis method.

On the other hand, Patent Document 1 describes that a value of S100P (one of the S100 protein family) included in at least one of pancreatic juice and a body fluid including pancreatic juice is higher in IPMN patients than benign pancreatic cyst patients.

### [Citation List]

### [Patent Literature]

[Patent Document 1] PCT International Publication No. WO 2012/137832

### [Summary of Invention]

### [Technical Problem]

While the method described in Patent Document 1 is useful for identifying an IPMN patient, a relation between the progress of IPMN and a value of S100P is unknown. Therefore, in follow-up examinations of IPMN patients, it is completely unknown whether measurement of S100P is useful for examining a transition of the progress of IPMN.

In view of the aforementioned circumstances, the inventors learned new knowledge about the value of S100P in IPMN patients and completed the present invention.

The present invention has been made in view of the aforementioned circumstances and provides a method through which it is possible to conveniently detect malignant pancreatic disease in IPMN patients without requiring advanced techniques.

### [Solution to Problem]

A method of determining pancreatic disease according to a first aspect of the present invention is a method of determining whether an IPMN patient have pancreatic disease. The method includes a first step of measuring a concentration of S100P in duodenal juice of the IPMN patient and a second step of determining at least one of IPMN malignancy and the presence of pancreatic cancer in the IPMN patient based on determination of whether the concentration of the S100P is equal to or greater than a predetermined cutoff value.

According to a second aspect of the present invention, in the first step, the duodenal juice may be treated using an inhibitor for protease that degrades the S100P.

### [Advantageous Effects of Invention]

According to the aspects, it is possible to provide a method of determining pancreatic disease through which it is possible to conveniently determine whether an IPMN patient has pancreatic disease.

### [Brief Description of Drawings]

Fig. 1 is a flowchart showing a flow of a method of determining pancreatic disease according to a first embodiment of the present invention.
Fig. 2 is a graph showing values of S100P in malignant IPMN patients and benign IPMN patients.
Fig. 3 is a graph showing values of S100P in IPMN follow-up patients.
Fig. 4 is a graph showing values of S100P in pancreatic cancer patients at an early stage and moderate degree.
Fig. 5 is a graph showing values (with treatment with an inhibitor) of S100P in malignant IPMN patients and benign IPMN patients.
Fig. 6 is a graph showing values (with treatment with an inhibitor) of S100P in IPMN follow-up patients.
Fig. 7 is a graph showing values (with treatment with an inhibitor) of S100P in pancreatic cancer patients at an early stage and moderate degree.

### [Description of Embodiments]

First, an overview of a method of determining pancreatic disease of the present invention will be described and a first embodiment will be then described.

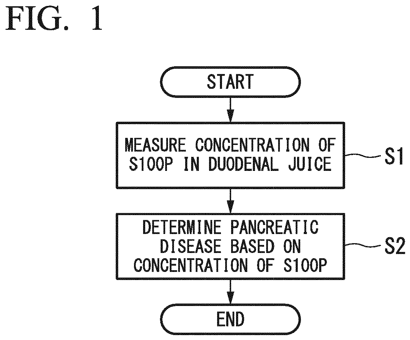

Fig. 1 is a flowchart showing a flow of a method of determining pancreatic disease of the present invention. The method of determining pancreatic disease includes Step S1 in which a concentration of S100P in duodenal juice of an IPMN patient is measured and Step S2 in which a pancreatic disease of a subject is determined based on the concentration of S100P.

In the first step (Step S1), duodenal juice of the IPMN patient is collected. The duodenal juice is a mixed body fluid of pancreatic juice discharged from the pancreas, bile discharged from the gall bladder or liver, mucus (intestinal juice) secreted from the duodenum, and the like. The method of collecting duodenal juice is not particularly limited. For example, duodenal juice can be easily collected through suction using an endoscope that is inserted into a body of a subject. The amount of a duodenal juice specimen may be, for example, about 0.1 to 0.5 milliliters (ml).

In the first step, the pancreatic juice is not isolated from the duodenal juice, and a concentration of S100P in the duodenal juice as the mixed body fluid is measured.

In the second step (Step S2), based on the determination of whether the measured value of S100P is equal to or greater than a predetermined cutoff value, it is determined whether the IPMN patient has pancreatic disease, and particularly, has malignant pancreatic disease such as a malignant IPMN or pancreatic cancer.

In IPMN patient groups, the degree of deviation in values of S100P and the relevancy between a value of S100P and a malignant pancreatic disease are totally unknown. However, the inventors found that, when an IPMN progresses to a malignant stage in IPMN patients, the value of S100P significantly increases.

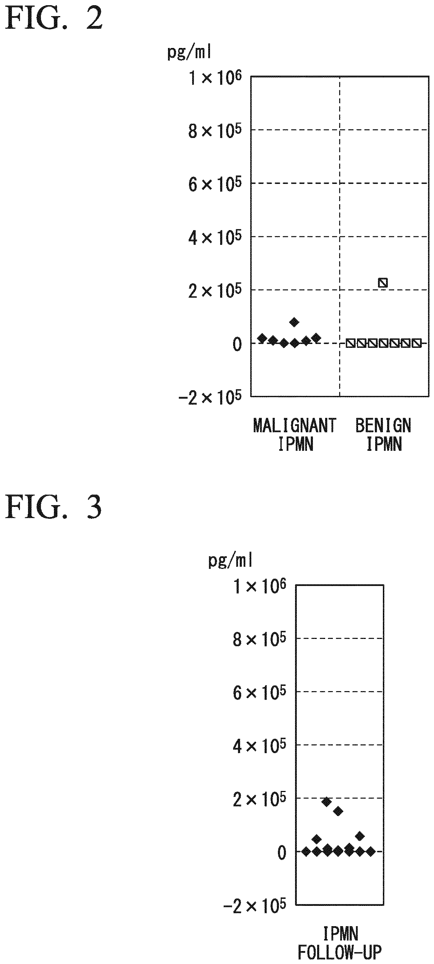

Fig. 2 shows values of S100P in duodenal juices collected from 7 malignant IPMN patients and values of S100P in duodenal juices collected from 8 benign IPMN patients. All of the 15 patients underwent surgery and it was checked whether an IPMN was diagnosed as being in a benign or malignant stage during the surgery.

In the 15 patients, when the cutoff value was set to 10000 pg/ml, the sensitivity of a malignant IPMN was 71.4% (5/7), a specificity of a malignant IPMN was 87.5% (7/8), the hit ratio of a malignant IPMN was 83.3% (5/6), the hit ratio of a benign IPMN was 77.8% (7/9), and the correct diagnosis rate was 80.0% (12/15), and it was possible to determine whether the stage was a benign or malignant stage with high accuracy.

Fig. 3 shows values of S100P in duodenal juices collected from 21 IPMN follow-up patients when take IPMN follow-up examinations. The 21 patients underwent accurate diagnostic imaging, and the 21 patients were determined as having an IPMN that had not progressed to a malignant stage. That is, all of the 21 patients were benign IPMN patients.

In the 21 IPMN follow-up patients, results were obtained such that, when a cutoff value was set to 10000 pg/ml, the specificity of a malignant IPMN and a correct diagnosis rate were 76.2% (16/21). Accordingly, when S100P was measured in IPMN patients, it was considered that benign and malignant stages are determined with high accuracy and it is possible to extract patients whose IPMN progressed to a malignant stage without performing accurate diagnostic imaging.

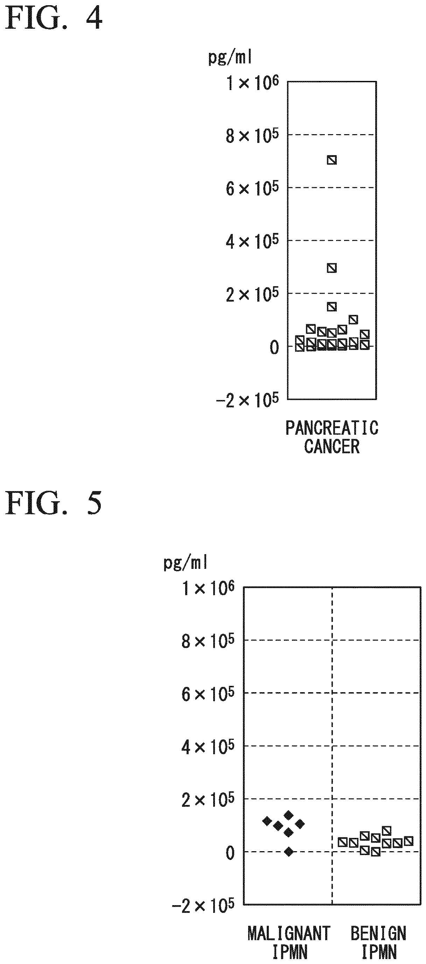

Pancreatic disease that can be screened according to the measurement of S100P in IPMN patients is not limited to IPMN malignancy. Fig. 4 shows values of S100P in duodenal juices collected from 37 pancreatic cancer patients at an early stage (stage 0 or I) and moderate degree (stages IIA and IIB). UICC classification (the sixth edition) was used as a staging system.

In the 37 pancreatic cancer patients, when a cutoff value was set to 10000 pg/ml, a sensitivity and correct diagnosis rate of 35.1% (13/37) were obtained. Based on the above result, the measurement of S100P was considered to be effective also in detecting pancreatic cancer in IPMN patients.

As described above, since the method of determining pancreatic disease of this embodiment includes the step (the second step) of determining pancreatic disease based on a concentration of S100P in duodenal juice, it is possible to determine pancreatic disease in an IPMN patient with high accuracy using a duodenal juice sample that is relatively easily available.

Next, a second embodiment of the present invention will be described. In this embodiment, in the first step, duodenal juice is treated with an inhibitor inhibiting protease which degrades S100P, and S100P is measured. That is, in this embodiment, by using the aforementioned inhibitor, since a function of protease that degrades S100P is inhibited, it is possible to reduce degradation of S100P in the duodenal juice.

An inhibitor to be used is not particularly limited as long as it has an inhibitory action for protease that degrades S100P, and known inhibitors can be appropriately selected and used.

Fig. 5 shows values of S100P in duodenal juices collected from 7 malignant IPMN patients and values of S100P in duodenal juices collected from 11 benign IPMN patients. All of the 18 patients underwent surgery, and it was checked whether an IPMN was diagnosed as being in a benign or malignant stage during the surgery. Note that the 18 patients were included in a group separate from the 15 patients of the first embodiment.

In the 18 patients, when the cutoff value was set to 73000 pg/ml, the sensitivity of a malignant IPMN was 87.5% (7/8), the specificity of a malignant IPMN was 81.8% (9/11), the hit ratio of a malignant IPMN was 87.5% (7/8), the hit ratio of a benign IPMN was 90.0% (9/10), and the correct diagnosis rate was 83.3% (15/18), and it was possible to determine a benign and malignant stage with higher accuracy than in the first embodiment.

Even when a cutoff value was set to 104000 pg/ml, the sensitivity of a malignant IPMN was 62.5% (5/8), the specificity of a malignant IPMN was 90.9% (10/11), the hit ratio of a malignant IPMN was 80.0% (4/5), the hit ratio of a benign IPMN was 76.9% (10/13), the correct diagnosis rate was 77.8% (14/18), and it was generally possible to determine whether the stage was a benign or malignant stage with high accuracy.

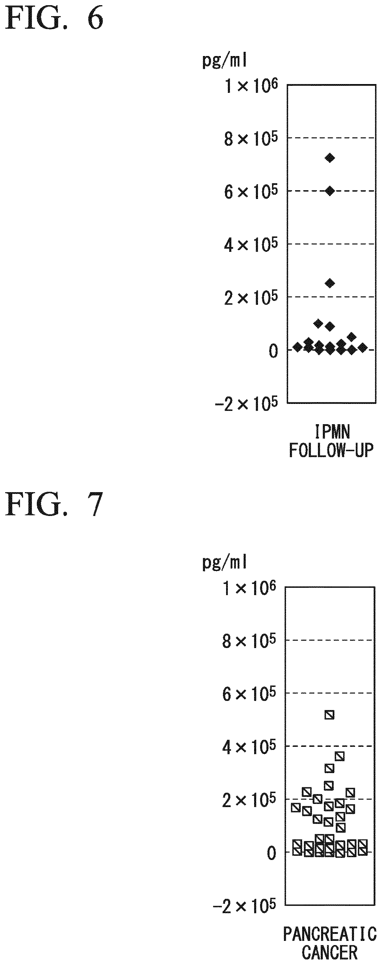

Fig. 6 shows values of S100P in duodenal juices collected from 17 IPMN follow-up patients. When the 17 patients underwent accurate diagnostic imaging, the 17 patients were determined as having an IPMN that had not progressed to a malignant stage. That is, all of the 17 patients were benign IPMN patients. Note that the 17 patients were included in a group separate from the 21 patients in the first embodiment.

In the 17 follow-up patients, when a cutoff value was set to 73000 pg/ml, the specificity of a malignant IPMN and the correct diagnosis rate were 70.6% (12/17), and the examination was considered to be performed with the same accuracy as in the first embodiment.

When a cutoff value was set to 104000 pg/ml, a specificity of a malignant IPMN and the correct diagnosis rate were 82.4% (14/17), and it was considered that pancreatic disease could be detected with higher accuracy than in the first embodiment.

Fig. 7 shows values of S100P in duodenal juices collected from 32 pancreatic cancer patients at an early stage and moderate degree. The staging system is similar to that in the first embodiment. Note that the 32 patients were included in a group separate from 37 patients in the first embodiment.

In the 32 pancreatic cancer patients, when the cutoff value was set to 73000 pg/ml, the sensitivity and the correct diagnosis rate were 50.0% (16/32), which were more favorable results than the first embodiment.

Even when a cutoff value was set to 104000 pg/ml, the sensitivity and the correct diagnosis rate were 46.9% (15/32), and favorable results were obtained.

As described above, also in the method of determining pancreatic disease in this embodiment, it is possible to determine pancreatic disease in an IPMN patient with high accuracy using a duodenal juice sample that is relatively easily available, similarly to the first embodiment.

In this embodiment, since a protease inhibitor is used to treat the duodenal juice in the first step, degradation of S100P is suppressed. As a result, even when a time is needed from when duodenal juice is collected until S100P is measured, since S100P is not degraded by protease, measurement accuracy can be maintained and accuracy itself can be expected to increase.

While the embodiments of the present invention have been described above, the scope of the present invention is not limited to the embodiments, and can be variously changed without departing from the spirit and scope of the present invention.

For example, a cutoff value of S100P in the method of determining pancreatic disease of the present invention is not limited to those of the aforementioned embodiments. Accordingly, the value may be appropriately set based on accumulated data and the like.

### [Industrial Applicability]

According to the embodiments, it is possible to provide a method of determining pancreatic disease through which it is possible to conveniently determine whether an IPMN patient has pancreatic disease.

### [Reference Signs List]

- S1: First step
- S2: Second step

## Claims

1. A method of determining pancreatic disease that is a method of determining whether an IPMN patient has pancreatic disease, the method comprising:
a first step of measuring a concentration of S100P in duodenal juice of the IPMN patient; and
a second step of determining at least one of IPMN malignancy and the presence of pancreatic cancer in the IPMN patient based on determination of whether the concentration of the S100P is equal to or greater than a predetermined cutoff value.

2. The method of determining pancreatic disease according to claim 1,
wherein, in the first step, the duodenal juice is treated using an inhibitor for protease that degrades the S100P.
